# EUROPÄISCHE PATENTSCHRIFT

(11) **EP 3 063 536 B1**
(45) Veröffentlichungstag und Bekanntmachung des Hinweises auf die Patenterteilung: **17.01.2018**
(21) Anmeldenummer: 14795970.4
(22) Anmeldetag: 21.10.2014
(51) Int. Cl.: G01N 27/30, G01N 27/44, G01N 33/18

(54) **ELEKTROCHEMISCHES VERFAHREN UND VORRICHTUNG ZUR BESTIMMUNG DES GEHALTS AN DOC UND/ODER TOC UND/ODER IC IN EINER WÄSSRIGEN PROBE**
ELECTROCHEMICAL METHOD AND DEVICE FOR THE DETERMINING THE CONTENT OF DOC AND/OR TOC AND/OR IC IN AN AQUEOUS SAMPLE
PROCÉDÉ ÉLECTROCHIMIQUE ET DISPOSITIF POUR LA DÉTERMINATION DE LA TENEUR EN DOC ET/OU TOC ET/OU IC DANS UN ÉCHANTILLON AQUEUX

(30) Priorität: 30.10.2013 AT 507072013
(43) Veröffentlichungstag der Anmeldung: 07.09.2016
(73) Patentinhaber: pro aqua Diamantelektroden Produktion GmbH & Co KG, 8712 Niklasdorf (AT)
(72) Erfinder: SCHELCH, Michael, A-8600 Oberaich (AT); STABER, Wolfgang, A-8600 Bruck an der Mur (AT); HERMANN, Robert, A-8600 Oberaich (AT); WESNER, Wolfgang, A-1220 Wien (AT)
(74) Vertreter: Vinazzer, Edith
(86) Internationale Anmeldenummer: PCT/EP2014/072510
(87) Internationale Veröffentlichungsnummer: WO 2015/062911

(56) Entgegenhaltungen:
- WO-A2-03/104765
- US-A- 5 672 516
- US-B1- 6 444 474

## Beschreibung

Die Erfindung betrifft ein Elektrochemisches Verfahren zur Bestimmung des Gehalts an DOC (Dissolved Organic Carbon) und/oder TOC (Total Organic Carbon) und/oder IC (Inorganic Carbon) in einer wässrigen Probe mittels anodischer Oxidation an Elektroden in einer geschlossenen Elektrolysezelle, wobei der Kohlenstoff zumindest zum Teil zu Kohlendioxid (CO₂) oxidiert wird. Die Erfindung betrifft ferner eine Vorrichtung mit einer geschlossenen Elektrolysezelle und zumindest einem CO₂-Sensor, zur Bestimmung des Gehalts an DOC (Dissolved Organic Carbon) und/oder TOC (Total Organic Carbon) und/oder IC (Inorganic Carbon) in einer wässrigen Probe mittels anodischer Oxidation an den Elektroden der Elektrolysezelle, wobei der CO₂-Sensor das gebildete CO₂ misst.

Der Bedarf an Messungen der Kohlenstoffparameter DOC (Dissolved Organic Carbon), TOC (Total Organic Carbon) und IC (Inorganic Carbon) zur Beurteilung der Qualität von Wasser, beispielsweise bei der Wasseraufbereitung in Kläranlagen, bei der Überwachung von industriellen Prozesswässern sowie im Naturraummonitoring ist hoch, gleichzeitig sind entsprechende Analysen im Verhältnis zur Analyse anderer Parameter aufwändig durchzuführen und relativ teuer. Die bekannten klassischen Verfahren basieren auf einer Verbrennung der Proben bei hohen Temperaturen von 700 °C bis 1000 °C auf Katalysatormaterialien, wie Ceroxid, im Sauerstoffstrom mit anschließender Detektion des entstehenden CO₂ mittels geeigneter Sensoren, beispielsweise IR-Detektoren. Alternativ dazu werden Geräte mit UV/Persulfat Aufschluss angeboten. Diese Verfahren benötigen große Mengen an Sauerstoff, da im Sauerstoffstrom gearbeitet wird, wobei die meisten Verfahren zusätzlich hohe Reaktionstemperaturen erfordern, woraus auch ein hoher Energiebedarf der Analysengeräte resultiert. Die diese Verfahren anwendenden Geräte nach dem Stand der Technik sind daher für eine mobile Anwendung kaum geeignet.

Es ist ferner bekannt, mittels anodischer Oxidation an Elektroden mit hoher Überspannung gegenüber der Sauerstoffentwicklung, beispielsweise Bleidioxidelektroden oder Bor dotierten Diamantelektroden, im Wasser gelöste organische Substanzen zu CO₂ zu oxidieren. Die DE 10 2008 010 581 A1 offenbart auf dieser Basis ein TOC-Messgerät, welches die als Nebenreaktion während der vollständigen Zersetzung der organischen Substanzen ablaufende elektrolytische Zerlegung von Wasser in H₂ und O₂ als Quelle für einen Trägergasstrom nutzt, welcher das gebildete CO₂ zum Sensor transportiert. Dieses Verfahren erfordert daher keine externe Sauerstoffquelle, benötigt jedoch bei relativ langer Laufzeit viel elektrische Energie, um einen kontinuierlichen Gasstrom aufrecht zu erhalten. Insbesondere sind auch hohe Anforderungen an den Sensor notwendig, da jeweils nur das gerade gebildete CO₂ im Gasstrom gemessen wird.

Der Erfindung liegt die Aufgabe zugrunde, ein Verfahren und eine Vorrichtung der eingangs genannten Art zur Verfügung zu stellen, welche eine mobile und besonders benutzerfreundliche Messung ermöglichen. Der Stromverbrauch soll moderat sein und neben Netzstrom auch mit handelsüblichen Batterien bzw. Akkumulatoren gedeckt werden können. Die Probenbehälter sollen ein Handling auch im Freien erleichtern und eine Kontamination von wässrigen Proben durch vorhergegangene Messungen ausschließen. Es sollen keine Chemikalien zum Einsatz kommen, welche eine besondere Entsorgung erfordern. Kurze Messzeiten und minimale Probenvorbereitung sollen gut reproduzierbare Werte liefern. Die Messvorrichtung soll klein, gewichtsmäßig leicht, gleichzeitig aber robust und auch kostengünstig ausgeführt werden können.

Was das Verfahren betrifft, wird die gestellte Aufgabe erfindungsgemäß dadurch gelöst, dass das an der Anode gebildete Kohlendioxid in einem geschlossenen Gasraum gesammelt wird, wobei im Bereich des im Wesentlichen linearen Anstiegs des CO₂-Gehaltes im Gasraum die Geschwindigkeit, mit der dieser Anstieg erfolgt, ermittelt wird und in einer Auswerteeinheit dazu herangezogen wird, den DOC-und/oder TOC- und/oder IC- Gehalt zu ermitteln.

Was die erfindungsgemäße Vorrichtung betrifft, wird die gestellte Aufgabe dadurch gelöst, dass separate Probenbehälter und ein externes Messgerät vorgesehen sind, wobei das Messgerät eine Aufnahme zum Einsetzen eines Probenbehälters aufweist und mit dem CO₂-Sensor versehen ist, welcher an der Innenseite eines am Messgerät drehbar gelagerten Elementes, insbesondere eines Deckels, positioniert ist, sodass er zur Bildung oder Mitbildung eines geschlossenen Gasraumes im Probenbehälter auf dem eingesetzten Probenbehälter positionierbar ist, wobei im Bereich des im Wesentlichen linearen Anstiegs des CO₂-Gehaltes im geschlossenen Gasraum die Geschwindigkeit, mit der dieser Anstieg erfolgt, ermittelt wird und in einer Auswerteeinheit dazu herangezogen wird, den DOC- und/oder TOC- und/oder IC-Gehalt zu ermitteln. Besonders vorteilhaft ist eine Ausführung des Probenbehälters mit einem Oberteil und einem die Elektrolysezelle beinhaltenden Unterteil.

Im Gegensatz zur DE 10 2008 010 581 A1 wird daher bei der gegenständlichen Erfindung das durch Elektrolyse gebildete CO₂ bei der Messung in einer geschlossenen Zelle, dem Gasraum, gesammelt, sodass sich dort die Konzentration von CO₂ unter Erhöhung des Gesamtdruckes während der Elektrolyse im Gasraum erhöht. Es wird daher stets die Summe des bis zum jeweiligen Zeitpunkt freigesetzten CO₂ gemessen. Das erfindungsgemäße Verfahren nützt die Tatsache, dass in einem geschlossenen Gasraum der CO₂-Gehalt derart ansteigt, dass nach einer Anfangsphase ein im Wesentlichen linearer Anstieg des CO₂-Gehaltes erfolgt, dessen "Steigung" zur Ermittlung des DOC- oder TOC-Gehaltes herangezogen wird. Da bei der Erfindung in Abhängigkeit von der Elektrolysedauer höhere Partialdrücke des gebildeten CO₂ auftreten, ist, verfahrensbedingt, bei gleicher Sensorqualität eine bessere Genauigkeit der Messung erzielbar. Dadurch vereinfacht sich auch der Aufbau des Messgerätes, da keine Leitungen, Öffnungen oder dergleichen vorgesehen werden müssen. Das in der Atmosphäre vorhandene CO₂ würde bei der aus der DE 10 2008 010 581 A1 offenbarten Anordnung gegebenenfalls stören, insbesondere wenn es über den Ausgang zum Sensor gelangt oder im Luftraum über der wässrigen Probe wäre. Da bei dem bekannten Verfahren das Messgas aus dem CO₂-freien Elektrolysegas plus dem gerade entstandenen CO₂ besteht, würden auch geringe Mengen an atmosphärischen CO₂ zu einem großen Fehler führen. Es müsste daher nicht nur verhindert werden, dass CO₂ vom Ausgang eindiffundiert, sondern es müssten auch der Reaktionsraum und das Leitungssystem vor der Messung CO₂ frei gemacht werden (Spülung, Bindung etc.). Bei der gegenständlichen Erfindung hingegen kann der Ausgangswert der CO₂ Konzentration vor Inbetriebnahme der Elektrolyse gemessen und abgezogen werden, bzw. es wird durch die erfindungsgemäße Auswertung des Anstiegs der CO₂ Konzentration der Absolutwert der Messung nicht benötigt.

Die Elektrolysezelle wird vorzugsweise mit einer Spannung betrieben, welche über der Wasserzersetzungsspannung liegt, um neben einer hohen Konzentration an Hydroxydradikalen auch Sauerstoff zu produzieren, welcher nicht nur zur schnelleren Bildung des CO₂-Gases aus den zu oxidierenden Kohlenstoffverbindungen beiträgt, sondern durch Blasenbildung auch das Ausgasen des gebildeten CO₂ unterstützt. Eine typische Spannung liegt für die zur Anwendung kommenden Elektroden über 2 V, insbesondere über 2,5 V.

Wie bereits erwähnt ist ein wesentlicher Vorteil des gegenständlichen Verfahrens, dass nicht auf die vollständige Aufoxidation des gesamten Kohlenstoffs in der wässrigen Probe gewartet wird, sondern die Geschwindigkeit der Kohlendioxidbildung, welche mit der Gesamtkonzentration des gelösten Kohlenstoffes in der Probe korreliert, herangezogen wird. Dies ermöglicht sehr kurze Analysezeiten und einen geringen Energiebedarf. Dabei entsteht in der kurzen Elektrolysezeit auch nur eine vergleichsweise geringe Gasmenge, welche im Gasraum unter mäßiger Druckerhöhung gesammelt wird.

Bei der erfindungsgemäßen Vorrichtung sind die Probenbehälter keine Komponenten des Messgerätes, sondern von diesem getrennt. Sie können mit der flüssigen Probe befüllt und ohne weiteres zwischengelagert werden. Besonders komfortabel gestaltet sich die Messung mit einer Ausführung des Probenbehälters mit Ober- und Unterteil, da bei dieser Ausgestaltung die flüssige Probe in den Unterteil eingebracht werden kann und anschließend das Oberteil, welches Teil des späteren Gasraum wird, aufgesetzt wird. Die elektrische Kontaktierung der Elektroden ist erst beim Positionieren im bzw. am Messgerät vorgesehen, der CO₂-Detektor ist ebenfalls im Messgerät untergebracht. Sowohl die Probenbehälter als auch das Messgerät können in einer sehr kompakten, daher mobilen und kostengünstigen Ausführung hergestellt werden.

Gemäß der Erfindung ist ferner vorgesehen, dass das in der Elektrolysezelle gebildete CO₂ vor dem Gasraum ein Trocknungsmittel, insbesondere ein Molekularsieb, durchläuft. Diese Maßnahme stellt sicher, dass sich im Gasraum ausschließlich CO₂ ansammelt und keine Feuchtigkeit aus der wässrigen Probe in den Gasraum gelangt.

Zur Ermittlung des TOC-Gehaltes einer wässrigen Probe werden die in der wässrigen Probe in schwer oxidierbarer Form vorhandenen Kohlenstoffverbindungen vor dem Einbringen der Probe in die Elektrolysezelle in gelösten Kohlenstoff umgewandelt. Diese Umwandlung kann auf einfache Weise und je nach Natur und Herkunft der wässrigen Probe durch entsprechende Aufschlusschemikalien, wie Säuren, Laugen oder hydrierende Substanzen, durchgeführt werden.

Alternativ können durch ein Filtrieren der wässrigen Probe vor ihrem Einbringen in die Elektrolysezelle partikuläre Kohlenstoffverbindungen aus der wässrigen Probe entfernt werden.

Besonders komfortabel ist ferner die Handhabung von Probenbehältern, wenn der Unterteil als länglicher, beispielsweise hohlzylindrischer Körper, ausgeführt ist, der oben offen und seitlich mit Öffnungen versehen ist, in welche die Elektroden von außen abdichtend eingesetzt sind und bei dem der Oberteil ebenfalls als länglicher, beispielsweise zylindrischer Körper ausgebildet ist, der auf den Unterteil aufsetzbar ist und in seinem oberen Abschnitt einen nach oben offenen Raum enthält, welcher im Messgerät den Gasraum bildet bzw. mitbildet. Besonders vorteilhaft ist, dass solche Probenbehälter aus Kunststoff gefertigt werden können und zur Einmalverwendung vorgesehen werden können. Die Elektroden werden insbesondere als kleine Scheiben ausgeführt, die quasi in den Unterteil von außen abdichtend in die vorgesehenen Öffnungen hineingedrückt werden. Auf diese Weise können die Elektroden im Messgerät auch problemlos kontaktiert werden.

Das Messgerät kann vorteilhafterweise derart gestaltet werden, dass der CO₂-Sensor an einem am Messgerät beweglich gelagerten Teil angebracht wird, welcher bei eingesetztem Probenbehälter auf der Öffnung im Oberteil, diese dicht verschließend, positioniert werden kann, sodass der Sensor den Gasraum mitumschließt. Bei einer bevorzugten Ausführungsform der Erfindung ist der CO₂-Sensor an der Innenseite eines am Messgerät drehbar gelagerten Deckels positioniert.

Das Messgerät kann die zur Messung erforderliche Steuereinheit und vorzugsweise auch die zur Auswertung erforderliche Auswerteeinheit enthalten. Alternativ kann vorgesehen sein, die Messdaten vom Messgerät auf ein externes Gerät, wie ein Mobiltelefon, einen Tablet-PC und dergleichen zu übertragen. Am bzw. vom externen Gerät aus können die Steuerung der Messung und die Auswertung des Messergebnisses erfolgen.

Zur Bestimmung des IC-Gehaltes einer wässrigen Probe kann im Probenbehälter eine spezielle Anode verwendet werden, und zwar eine Anode mit einer sehr geringen Überspannung gegenüber der Sauerstoffentwicklung, beispielsweise aus Kupfer und/oder Silber, sodass diese Anode in einer phosphorsauren Lösung eine Gasentwicklung ohne DOC-Oxidation bewirkt. Die phosphorsaure Lösung kann beispielsweise durch ein Einbringen von Phosphorpentoxid-Pulver in die wässrige Probe bei bereits geschlossenem, in das Messgerät eingesetztem Probenbehälter eingebracht werden. Bei einer alternativen Ausführungsform kann die Anode der Elektrolysezelle eine äußere Schicht oder Folie aus einem sich bei anodischem Stromfluss auflösenden Metall, ebenfalls, wie bereits erwähnt, aus Kupfer und/oder Silber, aufweisen, wobei hinter dieser Schicht in einem kleinen Hohlraum bzw. Reservoir Phosphorsäure oder ein Phosphorsäure bildender Feststoff, beispielsweise Phosphorpentoxid, eingefüllt ist. Diese Substanz wird nach Auflösung der äußeren Schicht freigesetzt, sodass die Probe angesäuert wird. Die Ansäuerung setzt das anorganisch gebundene Kohlendioxid frei, dieses gelangt in den Gasraum, wo der Kohlendioxidanstieg erfindungsgemäß ermittelt wird.

Bei einer weiteren, vorteilhaften Ausführungsform der Erfindung ist die Anode mehrschichtig aufgebaut und weist eine Diamantelektrode oder eine Bleidioxidelektrode als Basisschicht und eine äußere Kupfer- und/oder Silberschicht und ein Reservoir mit Phosphorsäure oder einem Phosphorsäure bildenden Feststoff auf. Mit einer derart ausgeführten Anode ist eine zweistufige Analyse, und zwar zuerst die Messung des IC und anschließend die Messung des DOC/TOC in einem Analysengang möglich. Nach Auflösung der Kupfer- und/oder Silberschicht beginnt die dahinterliegende Diamantelektrode bzw. Bleidioxidelektrode bei hoher Überspannung mit der Oxidation der organischen Verbindung. In der ersten Phase der Analyse ist daher das freigesetzte CO₂ dem anorganischen Kohlenstoff zuordenbar, in der zweiten Phase dem DOC bzw. TOC.

Die erfindungsgemäße Vorrichtung kann ferner auch derart ausgeführt werden, dass die Probenbehälter und das Messgerät Bestandteile eines kontinuierlich und automatisiert arbeitenden Messsystems sind, welches insbesondere ein automatisches Probenahmesystem, eine automatische Abfüllung der Proben in die in einem Autosampler befindlichen Unterteile, ein automatischen Aufsetzen der Oberteile und eine automatische Positionierung im Messgerät umfasst.

Bei einer weiteren erfindungsgemäßen Ausführung des Messgerätes ist unterhalb oder seitlich der Aufnahme für den Probenbehälter im Messgerät zumindest eine Ultraschallsonde eingebaut, sodass Ultraschall das Austragen des gebildeten CO₂ unterstützen kann.

Weitere Merkmale, Vorteile und Einzelheiten der Erfindung werden nun anhand der Zeichnung, die ein Ausführungsbeispiel darstellt, näher beschrieben. Dabei zeigen
Fig. 1 und Fig. 2 Seitenansichten eines erfindungsgemäßen Probenbehälters,
Fig. 3a und Fig. 3b jeweils einen Längsschnitt durch die beiden Hauptbestandteile des Probenbehälters gemäß der Schnittlinie III-III der Fig. 2 und
Fig. 4 eine Ansicht eines Messgerätes.

In der nachfolgenden Beschreibung der Figuren beziehen sich Begriffe wie "oben", "unten", "senkrecht", "waagrecht" und dergleichen auf die in den Figuren dargestellten Positionen der betreffenden Bestandteile des Probenbehälters bzw. des Messgerätes.

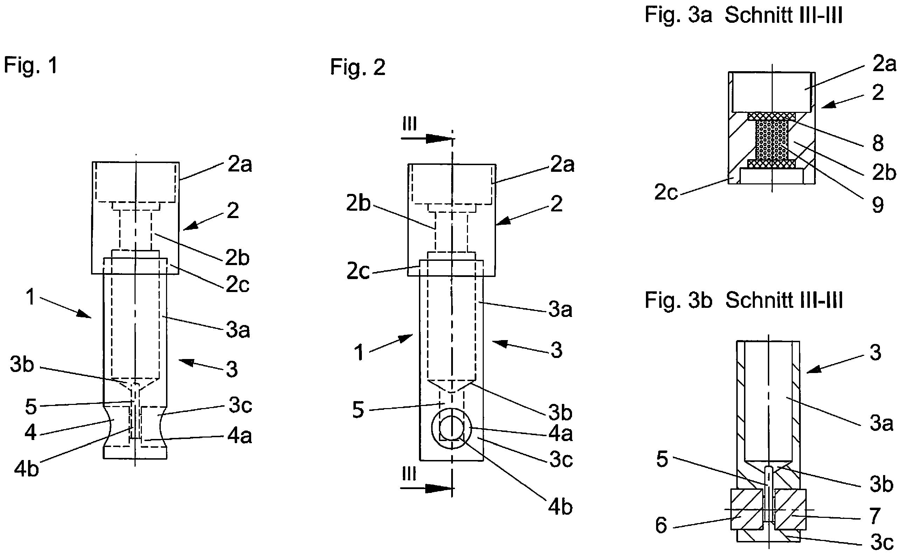

Die Figuren 1, 2, 3a und 3b zeigen eine Ausführungsform eines erfindungsgemäßen Probenbehälters 1. Der Probenbehälter 1 besteht aus einem Oberteil 2 und einem Unterteil 3, wobei der Oberteil 2 auf den Unterteil 3 abdichtend aufgesteckt bzw. aufgeschoben werden kann. Der Unterteil 3 ist ein oben offener, mit einem Boden versehener, im Wesentlichen zylindrischer Körper, welcher insbesondere eine Höhe von einigen Zentimetern, beispielsweise in der Größenordnung von 4 cm bis 6 cm, und einen größten Durchmesser von 10 mm bis 25 mm, insbesondere in der Größenordnung von 15 mm, aufweist. Der Unterteil 3 weist einen oberen, hohlzylindrischen Abschnitt 3a auf, welcher über eine konische Verengung 3b in einen unteren Abschnitt 3c übergeht, welcher, bis auf eine waagrechte Bohrung 4 und einen mittig, senkrecht verlaufenden, im Querschnitt langlochförmigen bzw. im Wesentlichen ovalen Verbindungskanal 5, aus Vollmaterial besteht. Der Verbindungskanal 5 verbindet die Bohrung 4 mit dem Hohlraum im oberen Abschnitt 3a. Bei der gezeigten Ausführungsform weist ferner die Bohrung 4 äußere, am Unterteil 3 nach außen mündende Bohrungsabschnitte 4a auf, die einen etwas größeren Durchmesser besitzen als kurze, zentrale und in den Verbindungskanal 5 mündende Bohrungsabschnitte 4b. In die äußeren Bohrungsabschnitte 4a werden, wie es Fig. 3a zeigt, von außen Elektroden 6, 7 abdichtend eingesetzt, die bei der gezeigten Ausführungsform scheibenförmig ausgeführt sind und durch Presssitz oder mittels eines Klebers im Bohrungsabschnitt 4a gehalten sind. Die Anoden können Bleidioxidelektroden oder andere Elektroden mit hoher Überspannung gegenüber der Sauerstoffproduktion, vorzugsweise aber Diamantelektroden sein. In Frage kommen insbesondere Diamantfilmelektroden oder Volldiamantelektroden, wobei zu letzteren auch solche gehören, die in ein Trägermaterial eingebettete Volldiamantpartikel aufweisen. Die Leitfähigkeit der Diamanten wird durch eine Dotierung, insbesondere mit Bor, sichergestellt. Bei Diamantelektroden mit einem Trägermaterial kann dieses beispielsweise aus Kunststoff, Titan, Graphit, Blei und dergleichen bestehen. Als Kathode kann eine metallische Elektrode oder ebenfalls eine Diamantelektroden eingesetzt werden. Die in die Bohrungsabschnitte 4a eingesetzten Elektroden 6, 7 sind von außen gut elektrisch kontaktierbar. Der Unterteil 3 des Probenkörpers 1 bildet somit eine Elektrolysezelle.

Der Oberteil 2 ist ebenfalls ein vorzugsweise zylindrisch ausgeführter Körper, welcher einen hohlzylindrischen oberen Abschnitt 2a aufweist, der einen Gasraum zum Sammeln des in der Elektrolysezelle gebildeten CO₂, wie noch beschrieben wird, zur Verfügung stellt. Dieser Gasraum weist insbesondere ein Volumen von 1 cm³ bis 20 cm³ auf. Der Oberteil, welcher vorzugsweise eine Höhe von 20 mm bis 50 mm und einen Außendurchmesser von 14 mm bis 30 mm besitzt, weist ferner einen relativ kurzen unteren Abschnitt 2c auf, dessen Innendurchmesser an den Außendurchmesser des Unterteils 3 derart angepasst ist, dass der Oberteil 2 auf den Unterteil 3 mit leichtem Presssitz aufgesetzt werden kann. Zusätzlich kann eine Ringdichtung am Ober-oder Unterteil angebracht sein, beispielsweise eine Silikondichtung. Zwischen dem oberen Abschnitt 2a und dem unteren Abschnitt 2c befindet sich ein mittlerer Abschnitt 2b, in welchem bei der gezeigten Ausführungsform der Oberteil 2 eine größere Wandstärke besitzt als im oberen und im unteren Abschnitt 2a, 2c und welcher zum oberen Abschnitt 2a und zum unteren Abschnitt 2c jeweils von einem gasdurchlässigen, jedoch für Flüssigkeiten weitgehend undurchlässigen Element 8, insbesondere einer Membran bzw. einem gasdurchlässigen Gitter, begrenzt ist. Zwischen den beiden Elementen 8 ist ein Trocknungsmittel als Granulat bzw. Pulver 9 eingebracht, beispielsweise Kalziumchlorid, Phosphorpentoxid oder ein Zeolith-Molekularsieb. Der im oberen Abschnitt 2a gebildete Gasraum ist, solange keine Messung stattfindet, nach oben offen. Der Oberteil 2 und der Unterteil 3 sind, abgesehen von den eingesetzten Bestandteilen, aus Kunststoff gefertigt. Der Probenbehälter 1 ist vorzugsweise zur Einmalverwendung vorgesehen und wird nach der Durchführung einer Messung entsorgt bzw. es können, soweit möglich, Bestandteile einer Wiederverwertung zugeführt werden.

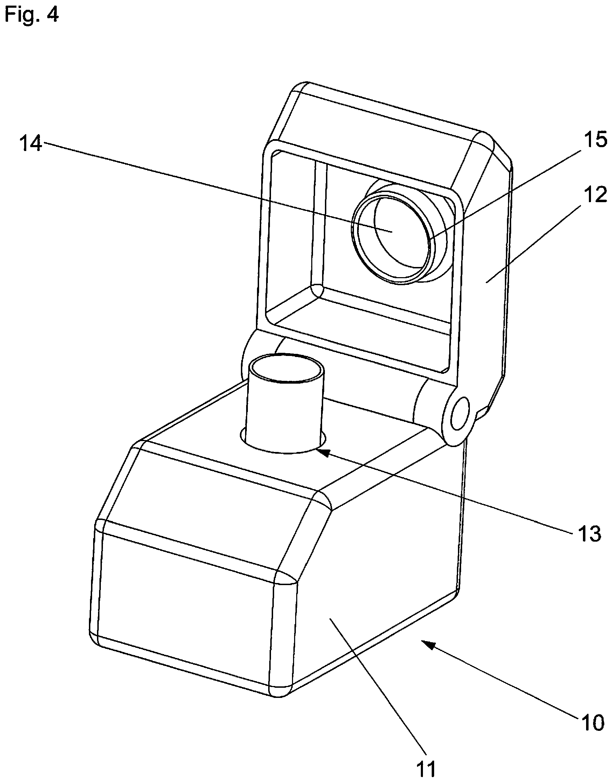

Fig. 4 zeigt schematisch eine Ausführungsform eines Messgerätes 10, welches gleichzeitig auch als Auswertegerät dienen kann. Das Messgerät 10 weist einen Basisteil 11 und einen Deckel 12 auf, welcher vorzugsweise am Basisteil 11 drehbar angeordnet ist. Im Basisteil 11 ist eine Aufnahme 13 für den Probenbehälter 1 eingebaut, in welche der Probenbehälter 1 von oben senkrecht einsetzbar ist und welche im Messgerätinneren die elektrische Kontaktierung der Elektroden 6, 7 sicherstellt. An der Deckelinnenseite ist zumindest ein CO₂-Sensor 14, beispielsweise ein IR-Detektor, eingebaut, derart, dass bei geschlossenem Deckel 12 ein den Sensorbereich umlaufendes, beispielsweise ringförmiges Verschlusselement 15 das luftdichte Verschließen des Gasraumes im oberen Abschnitt 2a des Oberteils 2 des eingesetzten Probenbehälters 1 gewährleistet.

Eine Steuer- und Auswerteeinheit mitsamt Anzeige des Messergebnisses kann im Messgerät 10 integriert sein. Alternativ kann vorgesehen sein, das Messgerät 10 mit einem Mobiltelefon, Tablet-PC oder einem sonstigen PC über Bluetooth, WLAN oder dergleichen zu verbinden, sodass unter Verwendung einer speziellen Applikationssoftware (einer APP) die Auswertung und Anzeige der Messergebnisses am jeweiligen externen Gerät möglich ist.

Unterhalb oder seitlich der Aufnahme 13 für den Probenbehälter 1 kann im Messgerät 10 zumindest eine nicht gezeigte, insbesondere handelsübliche Ultraschallsonde eingebaut werden, die im Basisteil 11 mit elektrischer Spannung versorgt wird.

Zur Ermittlung des DOC (Dissolved Organic Carbon-) Gehaltes einer wässrigen Probe, beispielsweise aus Abwasser, Wasser von Flüssen oder Seen, Trinkwasser, Badewasser, Grundwasser und dergleichen wird eine Flüssigkeitsprobe, welche optional einer Probenvorbereitung z.B. mittels Filtration, unterzogen wird, entnommen. Die Flüssigkeitsprobe wird nun in den aufrecht stehend positionierten Unterteil 3 des Probenbehälters 1 eingebracht und es wird anschließend der Oberteil 2 aufgesetzt. Der Probenbehälter 1 kann nun aufrecht stehend über längere Zeit gelagert werden. Zur Durchführung der Messung wird der Probenbehälter 1 in die Aufnahme 13 des Messgerätes 10 eingesetzt und es wird der Deckel 12 geschlossen. Durch Betätigen eines Schalters oder durch eine Auslösung über die Steuerungssoftware, auch von einem externen Gerät aus, wird die Elektrolysezelle in Betrieb genommen, das heißt die Elektroden 6, 7 werden mit Spannung versorgt. Diese Spannung muss in Abhängigkeit von der Zellgeometrie, der Leitfähigkeit der Probe und der Überspannung der Elektroden derart gewählt werden, dass eine elektrolytische Bildung von Sauerstoff / Wasserstoff zu beobachten ist. Vorzugsweise erfolgt die Regelung der Stromversorgung daher über die Regelung der Stromstärke. Die Stromstärke kann konstant gehalten werden, für spezielle Anwendungen kann durch Nutzung unterschiedlicher Stromstärken an den Elektroden die Oxidationskraft verändert werden. Diese Vorgangsweise ermöglicht die Unterscheidung leicht oxidierbarer von schwer oxidierbaren DOC Verbindungen. Die Stromstärke bestimmt bzw. beeinflusst die Elektrolysedauer und geht als Parameter in die Messauswertung ein. Der in der Flüssigkeitsprobe gelöste organische Kohlenstoff wird mittels anodischer Oxidation zumindest zum Teil zu CO₂ oxidiert, welches in den geschlossenen Gasraum im Oberteil 2 aufsteigt. Das Austragen des gebildeten CO₂ kann durch den von der Ultraschallsonde emittierten Ultraschall unterstützt werden. Die Ultraschallsonde kann separat oder automatisch in Betrieb genommen werden.

Im Gasraum erfolgt nach einiger Zeit, insbesondere in wenigen Minuten, nach Inbetriebnahme mit konstanter Stromstärke ein nahezu linearer Anstieg des CO₂-Gehaltes. Es wurde experimentell festgestellt, dass die Geschwindigkeit, mit der dieser Anstieg erfolgt, mit der Gesamtkonzentration an organischem Kohlenstoff in der wässrigen Probe korreliert. Durch Vergleich mit Daten (Anstieg des CO₂-Gehaltes im Gasraum über der Zeit), die mit Proben bekannter Kohlenstoffmengen ermittelt wurden, lässt sich daher aus der Geschwindigkeit, mit der die CO₂-Konzentration linear ansteigt, der tatsächliche Kohlenstoffgehalt ableiten bzw. errechnen. Dieser "Vergleich" erfolgt in der Software der Auswerteeinheit.

Es muss daher nicht, wie bei herkömmlichen DOC Messgeräten, auf die vollständige Oxidation des organischen Kohlenstoffs in der Probe gewartet werden. Dies ermöglicht kurze Analysezeiten, kleine Elektrodenflächen und einen geringen Energiebedarf. Es ist daher auch nur eine kurze Elektrolysezeit erforderlich und es entsteht eine vergleichsweise geringe Menge an Elektrolysegas, welches sich im Gasraum unter mäßiger Druckerhöhung sammelt. Grundsätzlich können daher auch größere Probenmengen verwendet werden als es bei den klassischen Verfahren der Fall ist, was eine repräsentative Probennahme und Messung erleichtert.

Für spezielle Fragestellungen, bzw. für die Überprüfung, ob bei bestimmten Proben der Anstieg wirklich mit dem Gesamtgehalt korreliert, ist es möglich, mit den Komponenten der erfindungsgemäßen Vorrichtung, eventuell unter Vergrösserung des Gasraumes, etwa durch entsprechende Ausgestaltung des Oberteils, oder durch eine Verringerung der Probenmenge zur Erweiterung des Messbereiches, eine vollständige Oxidation der Probe aufzuzeichnen und auszuwerten.

Um auch eine Messung des TOC (Total Organic Carbon)-Gehaltes durchzuführen, also eine Messung, die auch den Anteil an partikulärem Kohlenstoff in einer wässrigen Probe mitumfasst, wird in der Probe der partikuläre Kohlenstoff erfindungsgemäß vorab in gelösten Kohlenstoff umgewandelt. Diese Umwandlung kann je nach Natur und Herkunft der wässrigen Probe durch saure, basische oder hydrierende Aufschlüsse unter Verwendung nichtoxidativer Chemikalien erfolgen, optional unter Druckerhöhung, unter Erhöhung der Temperatur oder unter Einsatz von Katalysatoren. Anschließend kann die wässrige Probe, wie bereits beschrieben, gemessen werden. Diese Vorgehensweise hat sich als reproduzierbarer und zuverlässiger erwiesen als die gängigen oxidativen Aufschlüsse in UV-Verfahren, die gerade bei partikulären Substanzen, wie Biopolymeren und Kunststoffpartikeln, oft nur gering reproduzierbar sind. Der Aufschluss der wässrigen Probe erfolgt daher insbesondere außerhalb des Probenbehälters und auch außerhalb des Messgerätes und eröffnet daher die Möglichkeit, auch schwer aufschließbare Substanzen in gelösten Kohlenstoff umzuwandeln.

Um gegebenenfalls auch den IC-Gehalt einer wässrigen Probe bestimmen zu können, kann vorgesehen sein, in den Probenbehälter 1 eine Anode mit einer sehr geringen Überspannung gegenüber der Sauerstoffentwicklung, insbesondere aus Kupfer und/oder Silber, einzusetzen, welche in phosphorsaurer Lösung eine Gasentwicklung ohne DOC Oxidation bewirkt. Die phosphorsaure Lösung kann beispielsweise durch Injizieren von Phosphorsäure (H₃PO₄) in die wässrige Probe bei geschlossenem Probenbehälter 1 eingebracht werden. Alternativ kann die Anode der Elektrolysezelle eine äußere Schicht aus einem sich bei anodischem Stromfluss auflösenden Metall, wie beispielsweise Kupfer oder Silber, aufweisen, wobei hinter der Schicht in einem Hohlraum bzw. Reservoir Phosphorsäure oder ein Phosphorsäure bildender Feststoff, beispielsweise Phosphorpentoxid, eingefüllt ist, wobei diese Substanz nach Auflösung der Schicht freigesetzt wird, sodass die Probe angesäuert wird. Durch die Ansäuerung wird das anorganisch gebundene Kohlendioxid in den Gasraum freigesetzt, wo der äquivalente Kohlendioxidanstieg detektiert wird. Bei einer weiteren Ausführungsform der Erfindung kann die Anode mehrschichtig aufgebaut sein, mit einer Diamantelektrode oder einer Bleidioxidelektrode als Basisschicht und einer Kupfer- und/oder Silberschicht und einem Reservoir mit Phosphorsäure oder einem Phosphorsäure bildenden Feststoff. Eine derartige Anode gestattet eine zweistufige Analyse, zuerst die Messung des IC und anschließend die Messung des DOC/TOC in einem Analysengang. Erst nach der Auflösung der Kupfer- und/oder Silberschicht beginnt die dahinter liegende Diamantelektrode oder Bleidioxidelektrode bei hoher Überspannung mit der Oxidation der organischen Verbindungen. Dadurch kann das in der ersten Phase der Analyse freigesetzte CO₂ dem anorganischen Kohlenstoff zugeordnet werden. Die Zuordnung zu IC-Absolutwerten erfolgt wie die Zuordnung der DOC-Werte durch "Vergleich" mit geeigneten Standardlösungen in Form von Eichgeraden bzw. Eichkurven, wie dies auch bei den Methoden nach dem bisherigen Stand der Technik üblich ist.

Dies Probenbehälter 1 können auch Teil eines kontinuierlich arbeitenden Messsystems sein. Dabei werden die Proben durch ein automatisches Probenahmesystem gezogen und in die sich in einem Autosampler befindlichen Unterteile 3 eingefüllt. Das Aufsetzen der Oberteile 2 erfolgt ebenso automatisch wie die Positionierung des Probenbehälters 1 im Messgerät 10.

Die Erfindung ist auf das dargestellte Ausführungsbeispiel nicht eingeschränkt. So ist es beispielsweise möglich, den Probenkörper abweichend von einer zylindrischen Form, beispielsweise in Quaderform, zu gestalten.

**Bezugsziffernliste**
- 1: Probenbehälter
- 2: Oberteil
- 2a: oberer Abschnitt
- 2b: mittlerer Abschnitt
- 2c: unterer Abschnitt
- 3: Unterteil
- 3a: oberer Abschnitt
- 3b: Verengung
- 3c: unterer Abschnitt
- 4: Bohrung
- 4a: Bohrungsabschnitt
- 4b: zentraler Bohrungsabschnitt
- 5: Verbindungskanal
- 6, 7: Elektroden
- 8: Membran oder Gitter
- 9: Molekularsieb
- 10: Auswertegerät
- 11: Basisteil
- 12: Deckel
- 13: Aufnahme
- 14: Sensor
- 15: ringförmiges Verschlusselement

## Patentansprüche

1. Elektrochemisches Verfahren zur Bestimmung des Gehalts an DOC (Dissolved Organic Carbon) und/oder TOC (Total Organic Carbon) und/oder IC (Inorganic Carbon) in einer wässrigen Probe mittels anodischer Oxidation an Elektroden in einer geschlossenen Elektrolysezelle, wobei der Kohlenstoff zumindest zum Teil zu Kohlendioxid (CO₂) oxidiert wird,
**dadurch gekennzeichnet,**
**dass** das gebildete Kohlendioxid in einem geschlossenen Gasraum gesammelt wird, wobei im Bereich des im Wesentlichen linearen Anstiegs des CO₂-Gehaltes im Gasraum die Geschwindigkeit, mit der dieser Anstieg erfolgt, ermittelt wird und in einer Auswerteeinheit dazu herangezogen wird, den DOC- und/oder TOC- und/oder IC-Gehalt zu ermitteln.

2. Verfahren nach Anspruch 1, **dadurch gekennzeichnet, dass** als Anode in der Elektrolysezelle eine Anode mit Überspannung gegenüber der Sauerstoffbildung verwendet wird, insbesondere eine dotierte Diamantelektrode oder eine Bleidioxidelektrode, und dass im Betrieb das Potential der Überspannung überschritten wird, sodass neben OH-Radikalen gasförmiger Sauerstoff gebildet wird.

3. Verfahren nach Anspruch 1, **dadurch gekennzeichnet, dass** das in der Elektrolysezelle gebildete CO₂ vor dem Gasraum ein Trocknungsmittel, insbesondere ein Molekularsieb (9), durchläuft.

4. Verfahren nach einem der Ansprüche 1 bis 3, **dadurch gekennzeichnet, dass** in der wässrigen Probe vor ihrem Einbringen in die Elektrolysezelle partikuläre, in schwer oxidierbarer Form vorhandene Kohlenstoffverbindungen in gelösten Kohlenstoff umgewandelt werden, wobei die Umwandlung vorzugsweise mittels Aufschlusschemikalien, wie Säuren, Laugen, hydrierenden Substanzen, durchgeführt wird.

5. Verfahren nach einem der Ansprüche 1 bis 3, **dadurch gekennzeichnet, dass** die wässrige Probe vor ihrem Einbringen in die Elektrolysezelle filtriert wird.

6. Vorrichtung mit einer geschlossenen Elektrolysezelle und zumindest einem CO₂-Sensor (14), zur Bestimmung des Gehalts an DOC (Dissolved Organic Carbon) und/oder TOC (Total Organic Carbon) und/oder IC (Inorganic Carbon) in einer wässrigen Probe mittels anodischer Oxidation an den Elektroden der Elektrolysezelle, wobei der CO₂-Sensor (14) das gebildete CO₂ misst,
**dadurch gekennzeichnet,**
**dass** separate Probenbehälter (1) und ein externes Messgerät (10) vorgesehen sind, wobei das Messgerät (10) eine Aufnahme zum Einsetzen eines Probenbehälters (1) aufweist und mit dem CO₂-Sensor (14) versehen ist, welcher an der Innenseite eines am Messgerät (10) drehbar gelagerten Elementes, insbesondere eines Deckels (12), positioniert ist, sodass er zur Bildung oder Mitbildung eines geschlossenen Gasraumes im Probenbehälter (1) auf dem eingesetzten Probenbehälter (1) positionierbar ist, wobei im Bereich des im Wesentlichen linearen Anstiegs des CO₂-Gehaltes im geschlossenen Gasraum die Geschwindigkeit, mit der dieser Anstieg erfolgt, ermittelt wird und in einer Auswerteeinheit dazu herangezogen wird, den DOC- und/oder TOC- und/oder IC-Gehalt zu ermitteln.

7. Vorrichtung nach Anspruch 6, **dadurch gekennzeichnet, dass** der Probenbehälter einen Oberteil (2) und einen die Elektrolysezelle beinhaltenden Unterteil (3) aufweist, wobei der Unterteil (3) vorzugsweise als länglicher, beispielsweise hohlzylindrischer Körper ausgeführt ist, der oben offen ist und seitlich mit Öffnungen versehen ist, in welche die Elektroden (6, 7) von außen abdichtend eingesetzt sind.

8. Vorrichtung nach Anspruch 6 oder 7, **dadurch gekennzeichnet, dass** der Oberteil (2) als länglicher, beispielsweise zylindrischer Körper ausgebildet ist, der auf den Unterteil (3) aufsetzbar ist und in seinem oberen Abschnitt (2a) einen nach oben offenen Raum enthält, welcher den Gasraum bildet.

9. Vorrichtung nach einem der Ansprüche 6 bis 8, **dadurch gekennzeichnet, dass** im Oberteil (2) unterhalb des nach oben offenen Raumes ein Trocknungsmittel, insbesondere ein Molekularsieb (9), enthalten ist, wobei sich das Trocknungsmittel (9) vorzugsweise zwischen zwei Membranen und/oder Gittern (8) befindet, die für Gas durchlässig und für Flüssigkeit weitgehend undurchlässig sind.

10. Vorrichtung nach Anspruch 6, **dadurch gekennzeichnet, dass** das Messgerät (10) eine Steuereinheit, vorzugsweise auch die Auswerteeinheit, enthält.

11. Vorrichtung nach Anspruch 6, **dadurch gekennzeichnet, dass** die Messdaten vom Messgerät (10) auf ein externes Gerät, wie ein Mobiltelefon, ein Tablet-PC und dergleichen abrufbar und auf diesem speicherbar und weiter verarbeitbar sind, wobei vorzugsweise vom bzw. am externen Gerät die Steuerung der Messung und die Auswertung des Messergebnisses erfolgt.

12. Vorrichtung nach einem der Ansprüche 6 bis 11, **dadurch gekennzeichnet, dass** im Probenbehälter (1) eine Anode mit einer sehr geringen Überspannung gegenüber der Sauerstoffentwicklung, insbesondere aus Kupfer und/oder Silber, eingesetzt ist, welche in phosporsaurer Lösung eine Gasentwicklung ohne DOC Oxidation bewirkt.

13. Vorrichtung nach einem der Ansprüche 6 bis 11, **dadurch gekennzeichnet, dass** die Anode im Probenbehälter (1) eine äußere Schicht, die eine Kupfer- und/oder Silber- Elektrode ist, aufweist, wobei hinter dieser Schicht ein Reservoir vorhanden ist, welches einen phosphorsäurebildenden Stoff, vorzugsweise Phosphorpentoxid, enthält, wobei vorzugsweise die Anode hinter dem Reservoir eine Schicht aus einer Diamantelektrode oder einer Bleidioxidelektrode aufweist.

14. Vorrichtung nach einem der Ansprüche 6 bis 13, **dadurch gekennzeichnet, dass** die Probenbehälter (1) und das Messgerät (10) Bestandteile eines kontinuierlich und automatisiert arbeitenden Messsystems sind, insbesondere mit einem automatisches Probenahmesystem, einer automatischen Abfüllung der Proben in die in einem Autosampler befindlichen Unterteile (3), einem automatischen Aufsetzen der Oberteile (2) und einer automatischen Positionierung im Messgerät (10).

15. Vorrichtung nach einem der Ansprüche 6 bis 14, **dadurch gekennzeichnet, dass** unterhalb oder seitlich der Aufnahme (13) für den Probenbehälter (1)im Messgerät (10) zumindest eine Ultraschallsonde eingebaut ist.

## Claims

1. Electrochemical method for determining the content of DOC (Dissolved Organic Carbon) and/or TOC (Total Organic Carbon) and/or IC (Inorganic Carbon) in an aqueous test specimen by means of anodic oxidation of electrodes in a closed electrolysis cell, wherein the carbon is oxidised, at least partially, to form carbon dioxide (CO₂), **characterised in that**
the formed carbon dioxide is collected in a closed gas compartment wherein, in the region where the CO₂ content rises substantially linearly in the gas compartment, the speed at which this rise takes place is determined and is fed into an evaluation unit in order to determine the content of the DOC and/or TOC and/or IC.

2. Method according to claim 1, **characterised in that** an anode with an overvoltage with respect to the oxygen formation, in particular a doped diamond electrode or a lead dioxide electrode is used as an anode in the electrolysis cell and that, during operation, the potential of the overvoltage is exceeded so that, besides OH radicals, gaseous oxygen is formed.

3. Method according to claim 1, **characterised in that** the CO₂ formed in the electrolysis cell passes through a desiccant, in particular a molecular sieve (9), before reaching the gas compartment.

4. Method according to any one of claims 1 to 3, **characterised in that** particular carbon bonds in the aqueous test specimen, present in a form difficult to oxidise before their introduction into the electrolysis cell, are converted into dissolved carbon, wherein the conversion is performed preferably by means of decomposition chemicals such as acids, alkalis, and hydrating substances.

5. Method according to any one of claims 1 to 3, **characterised in that** the aqueous test specimen is filtered before introducing it into the electrolysis cell.

6. Device with a closed electrolysis cell and at least one CO₂ sensor (14), for determining the content of DOC (Dissolved Organic Carbon) and/or TOC (Total Organic Carbon) and/or IC (Inorganic Carbon) in an aqueous test specimen by means of anodic oxidation of the electrolysis cell, wherein the CO₂ sensor (14) measures the formed CO₂, **characterised in that**
separate specimen containers (1) and an external measuring device (10) are provided wherein the measuring device (10) has a receptacle into which a specimen container (1) can be inserted and is provided with the CO₂ sensor (14) which is positioned on the inner side of an element, in particular a cover (12), mounted rotatably on the measuring device (10) so that it can be positioned on the specimen container (1) in use to form or jointly form a closed gas compartment in the specimen container (1) wherein, in the region where the CO₂ content rises substantially linearly in the closed gas compartment, the speed at which this rise takes place is determined and is fed into an evaluation unit in order to determine the content of the DOC and/or TOC and/or IC.

7. Device according to claim 6, **characterised in that** the specimen container has an upper part (2) and a lower part (3) containing the electrolysis cell, wherein the lower part (3) is designed preferably as an elongated, for example hollow cylindrical body which is open at the top and provided with openings at the side in which the electrodes (6, 7) can be inserted from the outside forming seals.

8. Device according to claim 6 or 7, **characterised in that** the upper part (2) is formed as an elongated, for example hollow cylindrical body which can be placed on the lower part (3) and contains in its upper section (2a) a space, open at the top, which forms the gas compartment.

9. Device according to any one of claims 6 to 8, **characterised in that**, in the upper part (2) below the space open at the top, a desiccant, in particular a molecular sieve (9), is contained wherein the desiccant (9) is located preferably between two diaphragms and/or grids (8) which allow gas to pass through and are largely impervious for fluids.

10. Device according to claim 6, **characterised in that** the measuring device (10) contains a control unit, preferably the evaluation unit also.

11. Device according to claim 6, **characterised in that** the measurement data can be retrieved from the measuring device (10) to an external device, such as a mobile telephone, a tablet computer and similar and can be stored on them and processed further, wherein preferably the control of the measuring and evaluating of the measurement results can be carried out by or, respectively on the external device.

12. Device according to any one of claims 6 to 11, **characterised in that**, in the specimen container (1), an anode with a very small overvoltage with respect to the growth of the oxygen, is used, made in particular from copper and/or silver, and which causes gas to develop in a phosphoric acid solution without DOC oxidation.

13. Device according to any one of claims 6 to 11, **characterised in that** the anode in the specimen container (1) has an outer layer which is a copper and/or silver electrode, wherein, below this layer, a reservoir is present which contains a substance forming phosphoric acid, preferably phosphorus pentoxide, wherein, preferably, the anode below the reservoir has a layer made from a diamond electrode or a lead dioxide electrode.

14. Device according to any one of claims 6 to 13, **characterised in that** the specimen container (1) and the measuring device (10) are component parts of a continuously working and automated measuring system, in particular with an automatic sampling system, the automatic loading of the specimens into the lower parts (3) located in an automatic sampler, the automatic placing of the upper parts (2) on top and the automatic positioning in the measuring device (10).

15. Device according to any one of claims 6 to 14, **characterised in that** at least one ultrasonic probe is incorporated underneath or to the side of the receptacle (13) for the specimen container (1) in the measuring device (10).

## Revendications

1. Procédé électrochimique pour la détermination de la teneur en DOC (carbone organique dissous) et/ou en TOC (carbone organique total) et/ou en IC (carbone inorganique) dans un échantillon aqueux au moyen d'une oxydation anodique sur des électrodes dans une cellule électrolytique fermée, le carbone étant oxydé au moins en partie en dioxyde de carbone (CO₂),
**caractérisé en ce que**
le dioxyde de carbone produit est collecté dans un espace gazeux fermé, la vitesse à laquelle l'augmentation linéaire essentiellement de la teneur en CO₂ dans l'espace gazeux, qui a lieu dans la plage de ladite augmentation, étant établie et utilisée dans une unité d'évaluation afin d'établir la teneur en DOC et/ou TOC et/ou IC.

2. Procédé selon la revendication 1, **caractérisé en ce qu'**une anode à surtension par rapport à l'oxygène produite est utilisée comme une anode dans la cellule électrolytique, notamment une électrode en diamant dopée ou une électrode en dioxyde de plomb, et **en ce que** le potentiel de la surtension est dépassé en service de sorte que de l'oxygène gazeux soit produit en plus de radicaux OH.

3. Procédé selon la revendication 1, **caractérisée en ce que** le CO₂ produit dans la cellule électrolytique passe avant l'espace gazeux un agent déshydratant, en particulier un tamis moléculaire (9).

4. Procédé selon l'une des revendications 1 à 3, **caractérisé en ce que**, dans l'échantillon aqueux avant de le placer dans la cellule électrolytique, des composés de carbone particulaires présents sous forme peu oxydable sont transformés en carbone dissout, la transformation étant réalisée de préférence au moyen de produits chimiques de cuisson tels que des acides, lessives alcalines, substances hydrogénantes.

5. Procédé selon l'une des revendications 1 à 3, **caractérisé en ce que** l'échantillon aqueux est filtré avant de le placer dans la cellule électrolytique.

6. Dispositif avec une cellule électrolytique fermée et au moins un capteur de CO₂ (14) pour déterminer la teneur en DOC (carbone organique dissous) et/ou en TOC (carbone organique total) et/ou en IC (carbone inorganique) dans un échantillon aqueux au moyen d'une oxydation anodique sur les électrodes de la cellule électrolytique, le capteur de CO₂ (14) mesurant le CO₂ produit, **caractérisé en ce que**
des récipients pour échantillon (1) et un appareil de mesure externe (10) sont prévus, ledit appareil de mesure (10) présentant un support pour loger un récipient pour échantillon (1) et étant prévu avec un capteur de CO₂ (14), lequel est positionné sur l'intérieur d'un élément monté pivotant sur l'appareil de mesure (10), en particulier un couvercle (12) de sorte qu'il puisse être positionné sur le récipient pour échantillon (1) pour produire ou coproduire un espace gazeux dans le récipient pour échantillon (1), la vitesse à laquelle l'augmentation linéaire essentiellement de la teneur en CO₂ dans l'espace gazeux, qui a lieu dans la plage de ladite augmentation, étant établie et utilisée dans une unité d'évaluation afin d'établir la teneur en DOC et/ou TOC et/ou IC.

7. Dispositif selon la revendication 6, **caractérisé en ce que** le récipient pour échantillon présente une partie supérieure (2) et une partie inférieure (3) comprenant la cellule électrolytique, la partie inférieure (3) étant réalisée de préférence comme un corps oblong par exemple cylindrique creux qui est ouvert en haut et prévu avec des ouvertures latérales dans lesquelles les électrodes (6, 7) sont insérées de manière étanche de l'extérieur.

8. Dispositif selon la revendication 6 ou 7, **caractérisé en ce que** la partie supérieure (2) est formée de préférence comme un corps oblong par exemple cylindrique creux qui peut être disposé sur la partie inférieure (3) et qui comprend, dans sa section supérieure (2a), un espace ouvert vers le haut formant l'espace gazeux.

9. Dispositif selon l'une des revendications 6 à 8, **caractérisé en ce qu'**un agent déshydratant, en particulier un tamis moléculaire (9) se trouve dans la partie supérieure (2) en dessous de l'espace ouvert vers le haut, ledit agent déshydratant (9) se trouvant de préférence entre deux membranes et/ou grilles (8) qui sont perméables aux gaz et dans l'ensemble imperméables aux liquides.

10. Dispositif selon la revendication 6, **caractérisé en ce que** l'appareil de mesure (10) contient une unité de commande, de préférence également l'unité d'évaluation.

11. Dispositif selon la revendication 6, **caractérisé en ce que** les données de mesure de l'appareil de mesure (10) peuvent être consultées sur un périphérique externe, tel un téléphone portable, une tablette et autres similaires, et sauvegardées et traitées ultérieurement sur ledit périphérique externe, la commande de la mesure et l'évaluation des résultats de mesure étant réalisées de préférence du ou sur le périphérique externe.

12. Dispositif selon l'une des revendications 6 à 11, **caractérisé en ce qu'**une anode avec une surpression très faible par rapport à l'oxygène produite, notamment en cuivre et/ou en argent, qui provoque une formation de gaz sans oxydation DOC dans la solution d'acide phosphorique, est utilisée dans le récipient pour échantillon (1).

13. Dispositif selon l'une des revendications 6 à 11, **caractérisé en ce que** l'anode dans le récipient pour échantillon (1) présente une couche extérieure qui est une électrode en cuivre et/ou en argent, derrière ladite couche se trouvant un réservoir qui contient une substance générant de l'acide phosphorique, de préférence du pentoxyde de phosphore, l'anode présentant de préférence
derrière le réservoir une couche formée d'une électrode en diamant ou d'une électrode en dioxyde de plomb.

14. Dispositif selon l'une des revendications 6 à 13, **caractérisé en ce que** le récipient pour échantillon (1) et l'appareil de mesure (10) sont des composants d'un système de mesure fonctionnant en continu et automatiquement, notamment avec un système d'échantillonnage automatique, un système de remplissage automatique des échantillons dans la partie inférieure (3) se trouvant dans un échantillonneur automatique, un système de dépose automatique de la partie supérieure (2) et un système de positionnement automatique dans l'appareil de mesure (10).

15. Dispositif selon l'une des revendications 6 à 14, **caractérisé en ce qu'**au moins une sonde à ultrasons est montée dans l'appareil de mesure (10) en dessous ou sur le côté du support (13) du récipient pour échantillon (1).
